# EUROPEAN PATENT SPECIFICATION

(11) **EP 0 778 749 B1**
(45) Date of publication and mention of the grant of the patent: **27.12.2000**
(21) Application number: 95930763.8
(22) Date of filing: 28.08.1995
(51) Int. Cl.: A61B 17/04, A61B 17/42

(54) **SURGICAL INSTRUMENT FOR TREATING FEMALE URINARY INCONTINENCE**
CHIRURGISCHES INSTRUMENT ZUR BEHANDLUNG VON URIN-INKONTINENZ BEI FRAUEN
INSTRUMENT CHIRURGICAL POUR LE TRAITEMENT DE L'INCONTINENCE URINAIRE FEMININE

(30) Priority: 30.08.1994 SE 9402872
(43) Date of publication of application: 18.06.1997
(73) Proprietor: ETHICON, INC., Sommerville, New Jersey 08876-0151 (US)
(72) Inventor: CLAREN, Jan, S-226 47 Lund (SE); ULMSTEN, Ulf, S-182 35 Danderyd (SE)
(74) Representative: Fisher, Adrian John
(86) International application number: SE9500964
(87) International publication number: WO9606567

(56) References cited:
- EP-A- 0 598 976
- WO-A-90/03766

## Description

The invention relates to a surgical instrument used for treating female urinary incontinence, i.e. incapacity of controlling the discharge of urine.

Urinary incontinence may be caused by a defect function in the tissue or ligaments connecting the vaginal wall with the pelvic muscles and pubic bone.

WO-A-9 003 766 describes a method for treating female urinary incontinence without the necessity of opening the abdomen, which would require hospital care for may be four days. In this method a tape is passed into the body at each side of the urethra and is implanted between the vaginal wall and the abdominal wall extending over the pubis. The tape is tightened in order to bring the vaginal wall and the urethra into correct position in relation to the pubis and is left in the body in order that fibrous tissue shall develop around the tape, said fibrous tissue functioning as a supporting ligament in the soft tissue. The tape is removed from the body when such fibrous tissue has developed, which takes about two months.

A surgical instrument of special construction is also proposed for use with this method, comprising a shank having a handle at one end thereof and a curved portion at the other end thereof said portion being intended to be passed into the body via the vagina and being dimensioned to extend from the inside surface of the vaginal wall over the back of the pubic bone to the outside of the abdominal wall .

The result obtained by such surgery is not always satisfactory due to the fact that fibrous tissue will not develop sufficiently since the soft tissue between the vaginal wall and the abdominal wall is in bad condition.

The object of the invention is to provide improved and simplified surgery with a considerably improved prognosis with regard to restoration of the urinary continence, and for this purpose the invention provides a surgical instrument of the kind referred to above having the features of claim 1.

The invention will be explained in more detail with reference to the accompanying drawings which disclose the surgical instrument according to the invention as well as several surgical steps when practising the method of the invention using said surgical instrument.

In the drawings
FIG. 1 is a side view of the surgical instrument in one embodiment thereof,
FIG. 2 is a plan view of the surgical instrument,
FIG. 3 is an enlarged fragmentary axial cross sectional view of a coupling of the instrument for attaching an exchangeable part thereof,
FIGS. 4 to 10 illustrate diagrammatically several surgical steps of the method according to the invention, and
FIG. 11 in the same way illustrates the final step of the method.

The surgical instrument comprises a cylindrical tubulary shank 10 having at one end thereof a handle 11 which forms two in opposite directions in a common plane projecting wings 12 and an opening 13. At the other end of the shank there is a socket 14 which is partly passed onto the shank and is soldered or brazed to the shank, a portion of the socket projecting from the shank at said other end thereof. A cylindrical shaft 15 is rotatably mounted in the shank and can be rotated manually by means of a knob 16 axially knurled at the outside surface thereof, which is mounted to one end of the shaft and is received by opening 13. The other end of the shaft forms a cylindrical portion 17 of smaller outside diameter than the shaft, which joins a portion 18 having external threads, a smooth end portion 19 of further reduced diameter joining the threaded portion 18, end portion 19 forming a guide pin at said other end of the shaft. Portions 18 and 19 are received in the portion of socket 14 projecting from the shank, and also a shoulder 20 projecting from the shank is received in said portion.

The surgical instrument as described so far is intended to be used several times and therefore should consist of a material which can be sterilized by autoclaving, e.g. of stainless steel.

The surgical instrument also includes an exchangeable and disposable element 21 which will be termed needle. It is attached to the shank at a straight portion at one end of the needle and extends over substantially a quarter of a circle to the other, free end thereof in order to follow substantially the profile of the pubis between the vagina and the abdominal wall. The needle has circular cross section and has a smooth, preferably polished outside surface. It tapers slightly towards the free end thereof where the needle forms a point 22 by being faceted but it can als be blunt-ended and have a transversely cut end. The practical use of the surgical instrument will show which embodiment is to be preferred. The disposable needle shall be made either of a tissue compatible plastics, such as polycarbonate, or of steel or a similar material.

For attachment of needle 21 to shank 10 the needle has at said one end thereof where the needle forms a straight portion to be received at said portion in socket 14, an axial blind hole extending from the end surface said hole having a threaded portion 23 and inwardly thereof a narrower, cylindrical portion 24. Guide pin 19 is dimensioned to be guidingly received by said latter portion when the threaded portion 18 for attaching needle 21 to the rest of the surgical instrument is screwed into threaded portion 23 of the blind hole by rotating shaft 15 by manual rotation of knob 16, the end surfaces of the shank and the needle being pressed against each other. The needle should be oriented in a predetermined rotational position in relation to the shank; it should project at right angles to the plane of handle 16, and this rotational position is secured by shoulder 20 on the shank being received in a mating recess 25 in the outside surface of the needle.

Portion 23 of needle 21 instead of being threaded can be dimensioned such that the threaded portion 18 of shaft 15 cuts a thread in the plastics of the needle when being screwed thereinto.

When the two parts of the surgical instrument are screwed together in the manner described they form a rigid unit which can be controlled with great precision at handle 11 when it is used for surgery by applying the method of the invention.

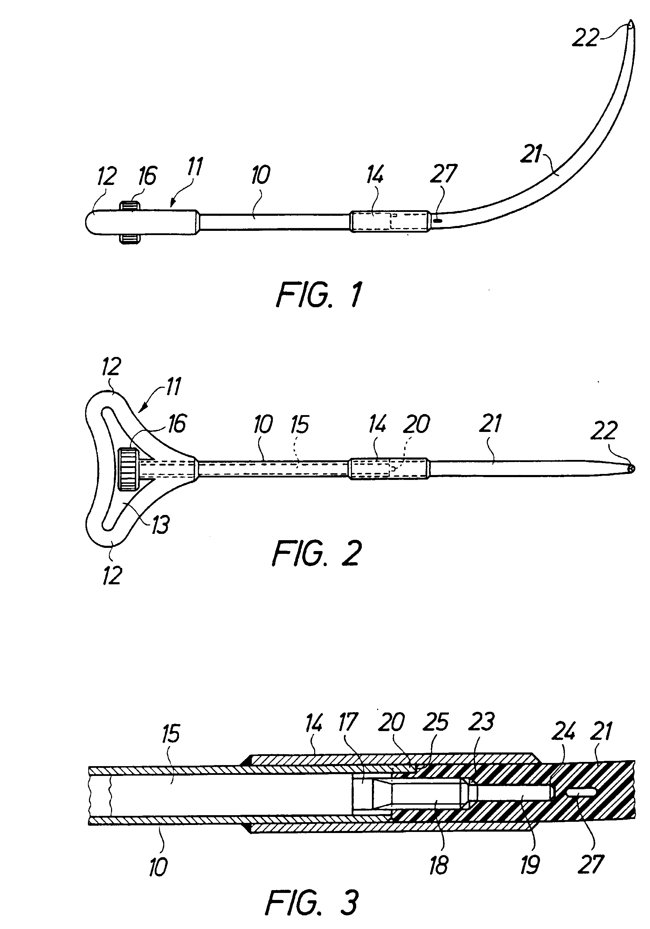

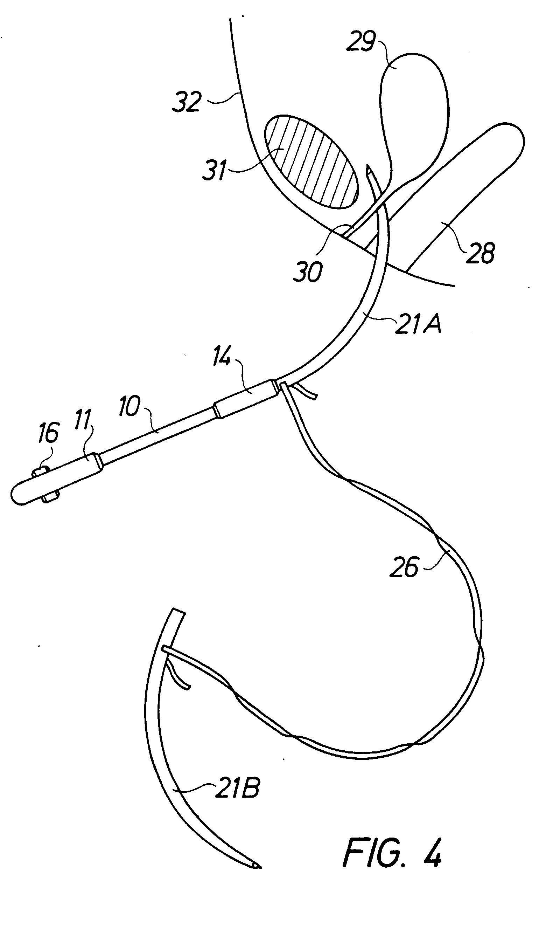

When the method according to the invention is practised two needles 21A and 21B of the embodiment described shall be connected one at each end of a tape 26, Fig. 4. In the preferred embodiment the tape end is glued to the needle but the connection can be effected also by the tape being passed through an eye 27, Fig. 3, in the needle adjacent the end attached to the shank or by the tape end being connected by ultrasonic welding to the needle or being baked into the plastics material of the needle at injection molding thereof.

The tape should be a woven tape having apertures between weft and warp of the order of 0.1 mm in order that fibroblasts shall be able to grow into the tape for anchoring of the tape in surrounding tissue. A suitable material for the tape is polypropylene which also can be coated with a fibroblast stimulating substance, e.g. an enamel matrix derivative. Preferably the tape has a width of 8 to 10 mm and a thickness of about 1 mm.

When the surgery for implanting the tape shall start one needle 21A is attached to shank 10, the other needle 21B hanging loosely in tape 26 as shown in Fig. 4.

In Figs. 4 to 11 the relevant parts of the female lower abdomen is disclosed diagrammatically, the vagina being designated 28, the urinary bladder 29, the urethra 30, the pubic bone 31, and the abdominal wall 32.

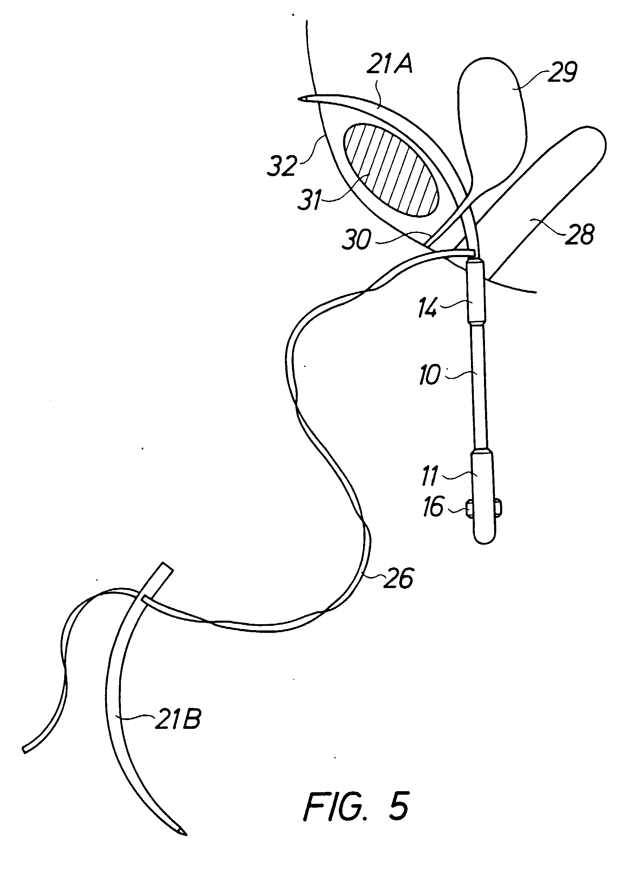

The first step of the surgery for implanting tape 26 is disclosed in Fig. 4 and comprises penetration of the vaginal wall by needle 21A a cut having first been made in said wall, and also penetration of the soft tissue at one side of urethra 30, the needle then according to Fig. 5 being passed close to the back of the pubic bone 31 and then through the abdominal wall above the pubic bone. A cut can be done through the abdominal wall for the passage of the needle therethrough but if the needle is pointed it may be sufficient to let the needle penetrate into the abdominal wall from the inside thereof and to make a registering cut in the abdominal wall on the outside thereof.

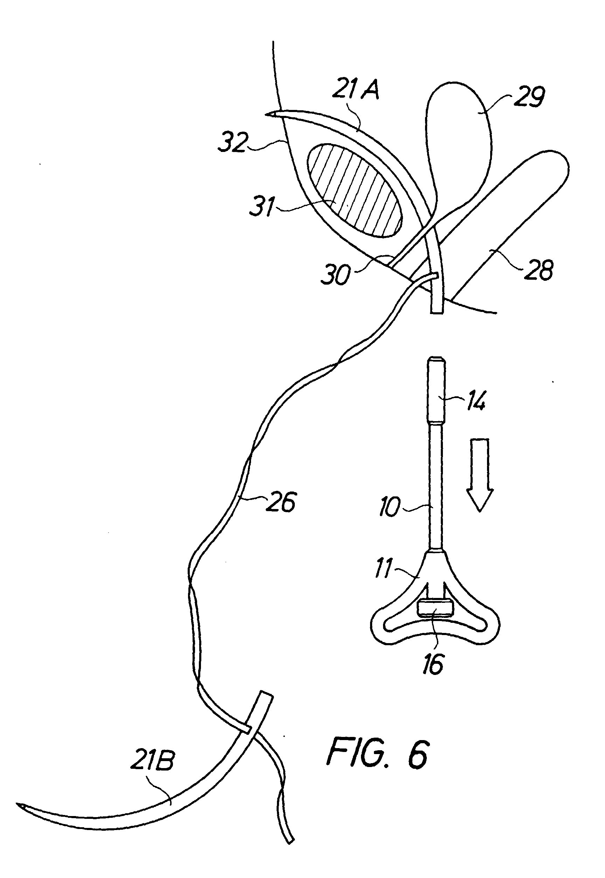

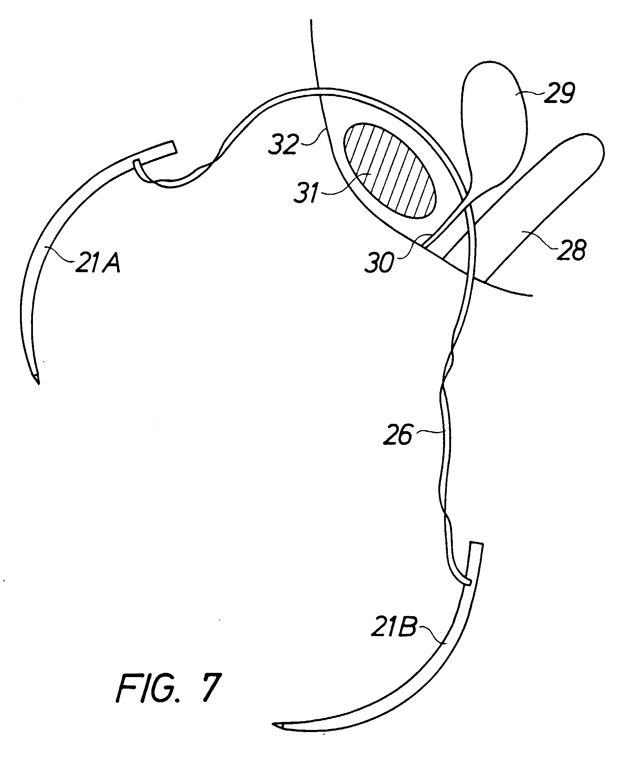

The shank of the instrument is now disconnected from needle 21A, Fig. 6, by rotating shaft 15 at knob 16 so that the threaded portion 18 of the shaft is unscrewed from the threaded portion 23 in needle 21A said needle then being withdrawn from the abdominal wall by means of forceps and tape 26 being pulled into and through the tissue as illustrated in Fig. 7.

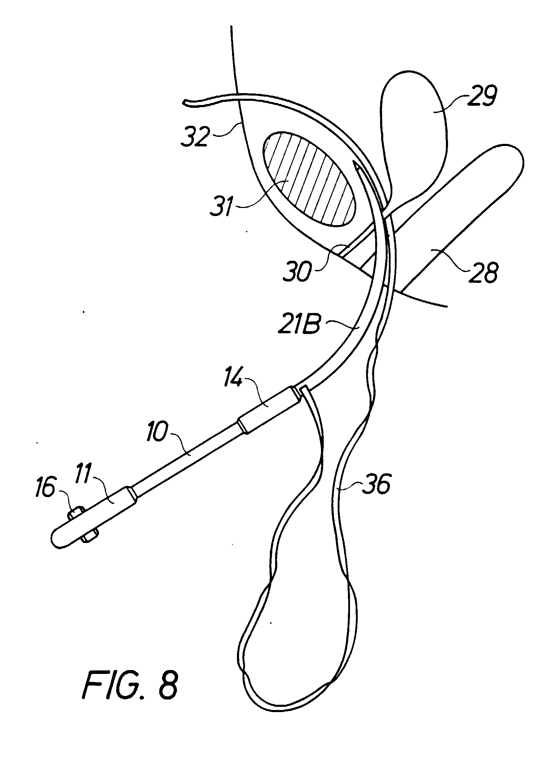

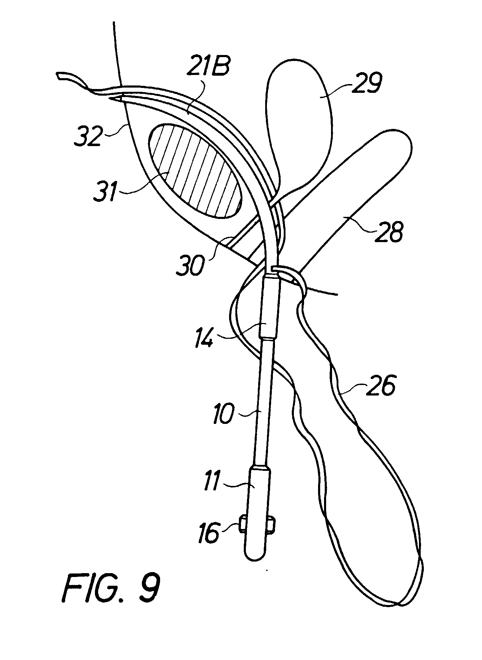

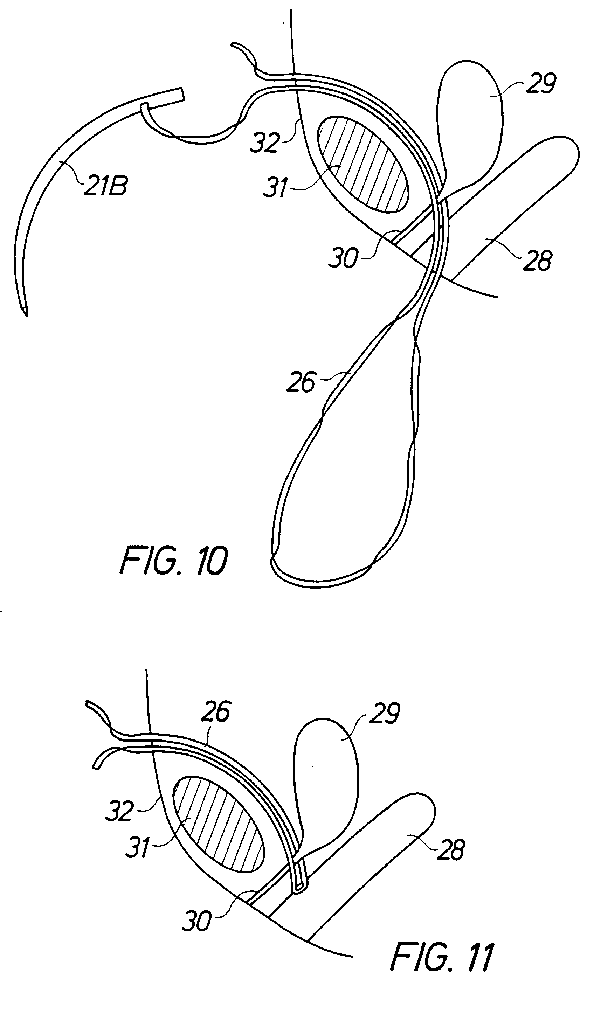

The other needle 21B is now attached to the shank, Fig. 8, and is passed through a cut in the vaginal wall to pass through the soft tissue at the other side of urethra 30. Needle 21B is passed through the abdominal wall, Fig. 9, and then, after having been disconnected from the shank, is withdrawn from the abdominal wall, Fig. 10, all in the same way as in the earlier procedure with neede 21A.

Tape 26 is now located at each side of urethra 30 as shown in Fig. 10 and is tightened with the loop formed by the tape located on the inside surface of the vaginal wall, Fig. 11. The surplus of the tape at the outside of the abdominal wall is cut off. Then, the tape is left as an implant in the body to form an artificial ligament attached to the abdominal wall and providing the support for urethra as required in order to restore the urinary continence.

Another kind of tape which may be used in the method according to the invention can be more closely woven than the tape mentioned above and can be of such material that the tape after a shorter or longer period will be completely resorbed. By the development of fibroblast proliferation stimulated by the tape the reinforcement of the tissue required in order to restore the urinary continence will be obtained.

## Claims

1. Surgical instrument for treating female urinary incontinence, comprising a shank (10) having a handle (11) at a first end of said shank, said shank being adapted at a second end of said shank to receive a first curved needle-like element (21A) or a second curved needle-like element (21B), said elements being constructed to be connectable independently of each other with the second end of said shank and said elements being intended to be passed into the body via the vagina and being dimensioned to extend from the inside surface of the vaginal wall over the back of the pubic bone to the outside of the abdominal wall, said first element being connected at one end of said first element to a first end of a tape (26) to be implanted into the body as a loop around the urethra, and said second element being connected at one end of said second element to a second end of said tape.

2. Instrument as in claim 1
**characterized** in that the shank (10) has a screw coupling (18, 23) for attachment of the element (21A, 21B) to the shank (10) .

3. Instrument as in claim 2
**characterized** in that the screw coupling comprises a shaft (15) rotatably mounted in the shank (10) and having an operating knob (16) at one end of the shaft said knob being available at the handle end of the shank, and a threaded portion (18) at the other end of the shaft for screw engagement with the element (21A, 21B).

4. Instrument as in any of claims 1 to 3
**characterized** in that the shaft (10) has a sleeve portion (14) at said other end thereof to receive therein an end portion of the needle-like element (21A, 21B) at said one end of the element.

5. Instrument as in any of claims 1 to 4
**characterized** in that the handle (11) comprises two wings (12) projecting diametrically from the shank (10).

6. Instrument as in claim 5
**characterized** in that the shank (10) and the needle-like elements (21A, 21B) have mutually co-operating means (20, 25) for positioning the respective elements on the shank (10) at right angles to the plane of the wings (12) .

7. Instrument as in any of claims 1 to 6
**chararacterized** in that the shank (10) is intended for use several times and consists of a material that can be autoclaved, the needle-like elements (21A, 21B) being intended for a single use and consist of plastics material, stainless steel or similar material.

8. Instrument as in claim 7
**characterized** in that the tape (26) is attached to the associated element (21A, 21B) by the tape ends being glued or welded to the elements or being baked into the plastics material of the elements.

9. Instrument as in claim 7
**characterized** in that the tape ends are passed through an eye (27) in the associated element (21A, 21B).

10. Instrument as in any of claims 1 to 9
**characterized** in that the needle-like elements are curved over substantially a quarter of a circle.

11. Instrument as in any of claims 1 to 10
**characterized** in that the elements (21A, 21B) taper towards the other, free end thereof.

12. Instrument as in claim 11
**characterized** in that said other end is pointed.

13. Instrument as in claim 11
**characterized** in that said other end is blunt.

14. Instrument as in any of claims 1 to 13
**characterized** in that the tape (26) is perforated for growth of fibroblasts thereinto.

15. Instrument as in claim 14
**characterized** in that the tape (26) comprises a woven tape.

16. Instrument as in claims 14 or 15
**characterized** in that the tape is coated with a fibroblast stimulating material.

## Patentansprüche

1. Chirurgisches Instrument zur Behandlung der Urin-Inkontinenz bei Frauen, umfassend einen Schaft (10), der an seinem einen Ende einen Handgriff (11) und an seinem anderen Ende einen gekrümmten Abschnitt (21) aufweist, wobei der Abschnitt (21) dafür vorgesehen ist, durch die Scheide in den Körper eingeführt zu werden und derart bemessen ist, daß er sich von der Innenseite der Scheidenwand hinter dem Schambein bis zur Außenseite der Bauchdecke erstreckt, dadurch gekennzeichnet, daß zwei gekrümmte, nadelförmige Elemente (21A, 21B), welche jeweils mit einem ihrer Enden mit den Enden eines Bandes (26) verbunden sind, welches als Schlinge rund um den Harnleiter permanent im Körper implantiert werden soll, derart aufgebaut sind, daß sie gleichzeitig mit dem Schaft (10) verbunden sind, um den gekrümmten Abschnitt zu bilden.

2. Instrument nach Anspruch 1, dadurch gekennzeichnet, daß der Schaft (10) eine Schraubkupplung (18, 23) zur Anbringung des Elementes (21A, 21B) am Schaft (10) aufweist.

3. Instrument nach Anspruch 2, dadurch gekennzeichnet, daß die Schraubkupplung eine Welle (15) umfaßt, welche drehbar im Schaft (10) montiert ist und an einem Ende einen am Handgriff-Ende des Schaftes zugänglichen Betätigungsknopf (16) sowie am anderen Ende einen Gewindeabschnitt (18) zum Einschrauben in das Element (21A, 21B) aufweist.

4. Instrument nach einem der Ansprüche 1 bis 3, dadurch gekennzeichnet, daß die Welle (10) an ihrem anderen Ende einen Hülsenabschnitt (14) zur Aufnahme der Endabschnitte an dem einen Ende des nadelförmigen Elementes (21A, 21B) aufweist.

5. Instrument nach einem der Ansprüche 1 bis 4, dadurch gekennzeichnet, daß der Handgriff (11) zwei Flügel (12) aufweist, die sich diametral von dem Schaft (10) aus erstrecken.

6. Instrument nach Anspruch 5, dadurch gekennzeichnet, daß der Schaft (10) und die nadelförmigen Elemente (21A, 21B) zusammenwirkende Elemente (20, 25) zum Positionieren des jeweiligen Elementes am Schaft im rechten Winkel zu den Flügeln (12) aufweisen.

7. Instrument nach einem der Ansprüche 1 bis 4, dadurch gekennzeichnet, daß der Schaft (10) für den mehrfachen Gebrauch vorgesehen ist und aus einem Material besteht, das im Autoklaven behandelt werden kann, während die nadelförmigen Elemente (21A, 21B) für den einmaligen Gebrauch bestimmt sind und aus einem Kunststoffmaterial, aus rostfreiem Stahl oder einem ähnlichen Material bestehen.

8. Instrument nach Anspruch 7, dadurch gekennzeichnet, daß das Band (26) am zugehörigen Element (21A, 21B) befestigt ist, indem die Enden des Bandes mit den Elementen verklebt, verschweißt oder im Kunststoffmaterial der Elemente eingeformt sind.

9. Instrument nach Anspruch 7, dadurch gekennzeichnet, daß die Enden des Bandes durch ein Öhr (27) am zugehörigen Element (21A, 21B) gefädelt sind.

10. Instrument nach einem der Ansprüche 1 bis 9, dadurch gekennzeichnet, daß die nadelförmigen Elemente (21A, 21B) im wesentlichen über einen Viertelkreis gekrümmt sind.

11. Instrument nach einem der Ansprüche 1 bis 10, dadurch gekennzeichnet, daß die Elemente (21A, 21B) zu ihrem anderen freien Ende hin verjüngt sind.

12. Instrument nach Anspruch 11, dadurch gekennzeichnet, daß das andere Ende angespitzt ist.

13. Instrument nach Anspruch 11, dadurch gekennzeichnet, daß das andere Ende stumpf ist.

14. Instrument nach einem der Ansprüche 1 bis 13, dadurch gekennzeichnet, daß das Band (26) perforiert ist, damit dort Fibroblasten einwachsen können.

15. Instrument nach Anspruch 14, dadurch gekennzeichnet, daß das Band (26) ein gewebtes Band ist.

16. Instrument nach Anspruch 14 oder 15, dadurch gekennzeichnet, daß das Band (26) mit einem die Ausbildung von Fibroblasten stimulierenden Material beschichtet ist.

## Revendications

1. Instrument chirurgical destiné au traitement de l'incontinence urinaire d'une femme, comportant une tige (10) ayant une poignée (11) à une première extrémité, et une partie incurvée (21) à son autre extrémité ladite partie étant prévue pour être passée à l'intérieur du corps via le vagin et étant dimensionnée pour s'étendre à partir de la surface intérieure de la paroi vaginale sur l'arrière de l'os pubien vers l'extérieur de la paroi abdominale, caractérisé en ce que deux éléments incurvés analogues à une aiguille (21A, 21B) qui sont chacun reliés à leur première extrémité à une première extrémité et à l'autre, respectivement, d'un ruban (26) à implanter de manière permanente dans le corps sous forme d'une boucle située autour de l'urètre, sont construits pour être reliés un à la fois à la tige (10) pour former ladite partie incurvée.

2. Instrument selon la revendication 1, caractérisé en ce que la tige (10) a un accouplement à vis (18, 23) destiné à la fixation de l'élément (21A, 21B) sur la tige (10).

3. Instrument selon la revendication 2, caractérisé en ce que l'accouplement à vis comporte un arbre (15) monté de manière rotative dans la tige (10) et ayant un bouton d'actionnement (16) à une première extrémité de l'arbre, ledit bouton étant disponible au niveau de l'extrémité de poignée de la tige, et une partie filetée (18) située à l'autre extrémité de l'arbre pour venir en prise vissée avec l'élément (21A, 21B).

4. Instrument selon l'une quelconque des revendications 1 à 3, caractérisé en ce que la tige (10) a une partie formant manchon (14) située à son autre extrémité pour recevoir dans celle-ci une partie d'extrémité de l'élément analogue à une aiguille (21A, 21B) située au niveau de ladite première extrémité de l'élément.

5. Instrument selon l'une quelconque des revendications 1 à 4, caractérisé en ce que la poignée (11) comporte deux ailes (12) faisant saillie diamétralement à partir de la tige (10).

6. Instrument selon la revendication 5, caractérisé en ce que la tige (10) et les éléments analogues à une aiguille (21A, 21B) ont des moyens coopérant mutuellement (20, 25) pour positionner les éléments respectifs sur la tige (10) selon des angles droits par rapport au plan des ailes (12).

7. Instrument selon l'une quelconque des revendications 1 à 6, caractérisé en ce que la tige (10) est prévue pour être utilisée plusieurs fois et est constituée d'un matériau qui peut être passé à l'autoclave, les éléments analogues à une aiguille (21A, 21B) étant prévus pour une utilisation unique et étant constitués d'une matière plastique, d'acier inoxydable ou d'un matériau similaire.

8. Instrument selon la revendication 7, caractérisé en ce que le ruban (26) est fixé à l'élément associé (21A, 21B) par les extrémités du ruban qui sont collées ou soudées sur les éléments ou agglutinées dans la matière plastique des éléments.

9. Instrument selon la revendication 7, caractérisé en ce que les extrémités de ruban sont passées à travers un oeillet (27) de l'élément associé (21A, 21B).

10. Instruments selon l'une quelconque des revendications 1 à 9, caractérisé en ce que les éléments analogues à une aiguille sont incurvés sur pratiquement un quart de cercle.

11. Instrument selon l'une quelconque des revendications 1 à 10, caractérisé en ce que les éléments (21A, 21B) sont effilés en direction de leur autre extrémité libre.

12. Instrument selon la revendication 11, caractérisé en ce que ladite autre extrémité est pointue.

13. Instrument selon la revendication 11, caractérisé en ce que ladite autre extrémité est émoussée.

14. Instrument selon l'une quelconque des revendications 1 à 13, caractérisé en ce que le ruban (26) est perforé pour la croissance de fibroblastes dans celui-ci.

15. Instrument selon la revendication 14, caractérisé en ce que le ruban (26) est constitué d'un ruban tissé.

16. Instrument selon la revendication 14 ou 15, caractérisé en ce que le ruban est revêtu d'un matériau stimulant les fibroblastes.
